# FASCICULE DE BREVET EUROPEEN

(11) **EP 4 237 050 B1**
(45) Date de publication et mention de la délivrance du brevet: **23.10.2024**
(21) Numéro de dépôt: 21815564.6
(22) Date de dépôt: 02.11.2021
(51) Int. Cl.: A61M 11/00, A61M 15/00, A61M 5/24, A61M 5/315

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 02.11.2020 FR 2011233
(43) Date de publication de la demande: 06.09.2023
(73) Titulaire: APTAR France SAS, 27110 Le Neubourg (FR)
(72) Inventeur: GOMEZ, Thomas, 69009 Lyon (FR); LE FAOU, Philippe, 69130 Ecully (FR); MGHIMIMI, Khaoula, 69130 Ecully (FR); TEKCE, Ege, 69340 Francheville (FR)
(74) Mandataire: CAPRI
(86) Numéro de dépôt international: PCT/FR2021/051915
(87) Numéro de publication internationale: WO 2022/090675

(56) Documents cités:
- WO-A1-2019/122671
- US-A- 5 893 484

## Description

La présente invention concerne un dispositif de distribution de produit fluide, notamment de type unidose ou bidose.

Plus particulièrement, la présente invention concerne un dispositif de distribution de produit fluide comportant une tête de distribution de produit fluide pour distribuer une ou deux dose(s) d'un produit fluide pharmaceutique. Dans ce type de dispositif, généralement appelé unidose ou bidose, chaque dose de produit fluide, généralement liquide, est distribuée ou pulvérisée à travers un orifice de distribution lors d'un actionnement manuel de ce dispositif. Si le dispositif est un unidose, la totalité du produit fluide est distribuée en un seul actionnement. Si le dispositif est un bidose, le produit fluide est distribuée en deux actionnements successifs du dispositif.

Généralement, ce type de dispositif unidose ou bidose est utilisé pour une distribution nasale, buccale ou sublinguale du produit fluide. Dans le cas d'une distribution nasale, la tête de distribution comporte alors une extension axiale adaptée à pénétrer dans une narine d'un utilisateur lors de l'utilisation.

Ce type de dispositif peut présenter certains inconvénients.

Ainsi, les dispositifs du type unidose ou bidose utilisent généralement une aiguille creuse, généralement métallique, pour percer le bouchon lors de l'actionnement. Or, l'utilisation d'une aiguille métallique est couteuse, et rend le recyclage du dispositif après utilisation plus complexe. De plus, lors du perçage du bouchon par l'aiguille, des particules d'élastomère peuvent se détacher du bouchon et polluer le fluide distribué.

Un autre problème se pose lorsqu'on utilise un bouchon-piston, qui au repos agit en tant que bouchon et lors de l'actionnement en tant que piston. Pour assurer une bonne étanchéité au repos, une interaction forte entre la surface radiale externe du bouchon-piston et la paroi cylindrique interne du réservoir est souhaitable, mais une telle interaction forte risque de gêner le déplacement du bouchon-piston lors de l'actionnement. De manière inverse, une faible interaction favorise un actionnement aisé, mais génère potentiellement des problèmes d'étanchéité au repos. Un bon compromis n'est pas facile à trouver, et en raison des tolérances de fabrication à la fois du bouchon-piston et du réservoir, la reproductibilité des propriétés n'est pas assurée.

Les documents US5893484 et WO2019122671 décrivent des dispositifs de l'état de la technique.

La présente invention a pour but de fournir un dispositif de distribution de produit fluide qui ne reproduit pas les inconvénients susmentionnés.

La présente invention a ainsi pour but de fournir un dispositif de distribution de produit fluide qui assure une parfaite étanchéité au repos ainsi qu'un actionnement aisé, indépendamment des tolérances de fabrication.

La présente invention a aussi pour but de fournir un dispositif de distribution de produit fluide qui évite tout risque de génération de particules d'élastomère lors de l'actionnement.

La présente invention a également pour but de fournir un tel dispositif de distribution de produit fluide qui soit simple et peu coûteux à fabriquer et à assembler.

La présente invention a donc pour objet un dispositif de distribution de produit fluide comportant un réservoir contenant au moins une dose de produit fluide, un bouchon-piston, monté coulissant dans ledit réservoir pour distribuer du produit fluide, une tête de distribution, pourvue d'un orifice de distribution, axialement déplaçable par rapport audit réservoir pour déplacer ledit bouchon-piston dans ledit réservoir pour ainsi distribuer du produit fluide à travers ledit orifice de distribution, ledit bouchon-piston comportant un manchon creux pourvu d'une zone de réception centrale disposée sensiblement au centre axial et radial dudit bouchon-piston, un élément d'obturation étant disposé dans ladite zone de réception centrale en position d'obturation et étant lors de l'actionnement expulsé de ladite position d'obturation vers une position d'ouverture, ladite zone de réception centrale se prolongeant axialement d'un côté par une zone de réception inférieure comportant au moins deux parties de paroi séparées par au moins deux rainures radiales s'étendant axialement, lesdites parties de paroi de ladite zone de réception inférieure retenant ledit élément d'obturation dans sa position d'ouverture, avec le produit fluide pouvant lors de l'actionnement s'écouler depuis ledit réservoir autour dudit élément d'obturation en position d'ouverture à travers lesdites rainures radiales vers ledit orifice de distribution.

Avantageusement, ledit bouchon-piston comporte une zone de réception supérieure comportant au moins deux parties de paroi séparées par au moins deux rainures radiales s'étendant axialement, ladite zone de réception supérieure étant symétrique par rapport à ladite zone de réception inférieure, de sorte que ledit bouchon-piston est symétrique et utilisable indifféremment dans un sens ou dans l'autre.

Avantageusement, chaque zone de réception a une forme légèrement conique s'évasant radialement vers l'extérieur à partir de la zone de réception centrale.

Avantageusement, le diamètre interne maximal desdites parties de paroi se situe au niveau du bord axial extérieur de chaque zone de réception supérieure et inférieure, ledit diamètre interne maximal étant sensiblement identique au diamètre externe maximal dudit élément d'obturation.

Avantageusement, chaque zone de réception comporte quatre parties de paroi et quatre rainures radiales réparties sur la périphérie.

Avantageusement, ledit bouchon-piston comporte une partie externe d'étanchéité, formée sur la périphérie externe dudit manchon creux, ladite partie d'étanchéité comportant au moins un bourrelet périphérique qui réalise l'étanchéité contre une paroi latérale interne dudit réservoir.

Avantageusement, ledit élément d'obturation, en position d'obturation, exerce une force radiale sur ladite partie externe d'étanchéité, augmentant l'interaction entre ledit bouchon-piston et ledit réservoir et donc l'étanchéité, et lors de l'actionnement, lorsque ledit élément d'obturation est expulsé de sa position d'obturation, l'interaction entre ledit bouchon-piston et ledit réservoir diminue, facilitant ainsi le déplacement dudit bouchon-piston dans ledit réservoir lors de l'actionnement.

Avantageusement, ledit élément d'obturation est sphérique, tel qu'une bille.

Avantageusement, ladite zone de réception centrale est définie entre un rétrécissement supérieur et un rétrécissement inférieur, de sorte qu'en position d'obturation, il y a deux surfaces périphériques d'étanchéité entre ledit élément d'obturation et ledit bouchon-piston.

Avantageusement, ladite tête de distribution comporte un canal de distribution reliant une pointe d'ouverture audit orifice de distribution, ladite pointe d'ouverture coopérant lors de l'actionnement avec ledit élément d'obturation pour le déplacer de sa position d'obturation vers sa position d'ouverture, ladite pointe d'ouverture comportant à son extrémité axiale une ou plusieurs ouvertures, tels que des trous ou des fentes, qui permettent le passage de produit fluide lorsque ladite pointe d'ouverture est en contact avec ledit élément d'obturation.

Avantageusement, ledit réservoir contient deux doses de produit fluide, distribuées lors de deux actionnements successifs.

En variante, ledit réservoir contient une seule dose de produit fluide, distribuée lors d'un seul actionnement.

Ces avantages et caractéristiques et d'autres de la présente invention apparaitront plus clairement au cours de la description détaillée suivante, faite en référence aux dessins joints, donnés à titre d'exemples non limitatifs, et sur lesquels
La figure 1 est une vue schématique en section transversale d'un dispositif de distribution de produit fluide de type bidose selon un mode de réalisation avantageux, en position de repos avant distribution de la première dose,
La figure 2 est une vue de détail en section transversale du bouchon-piston, sans l'élément d'obturation assemblé,
La figure 3 est une vue similaire à celle de la figure 2, avec l'élément d'obturation assemblé en position d'obturation,
La figure 4 est une vue similaire à celle de la figure 3, montrant la pointe d'ouverture avant expulsion de l'élément d'obturation de sa position d'obturation,
La figure 5 est une vue similaire à celle de la figure 4, après expulsion de l'élément d'obturation de sa position d'obturation vers sa position d'ouverture,
La figure 6 est une vue schématique en perspective du bouchon-piston, avec l'élément d'obturation en position d'obturation,
La figure 7 est une vue similaire à celle de la figure 3, avec l'élément d'obturation expulsé de sa position d'obturation et maintenu en position d'ouverture, et
La figure 8 est une vue schématique en perspective du bouchon-piston, avec l'élément d'obturation en position d'ouverture.

Dans la description ci-après, les termes "axial" et "radial" se réfèrent à l'axe central longitudinal du dispositif. Les termes "supérieur" et "inférieur" se réfèrent à la position droite du piston-bouchon représenté sur les figures.

La présente invention sera décrite ci-après en référence à un exemple de réalisation qui est un bidose, c'est-à-dire un dispositif contenant deux doses de produit fluide à distribuer lors de deux actionnements successifs du dispositif. Il est toutefois entendu que la présente invention pourrait s'appliquer à des dispositifs unidoses ne comportant qu'une seule dose, ou à des dispositifs contenant plus de deux doses, par exemple trois ou quatre doses. De même, le dispositif de type bidose représenté sur les dessins n'est qu'un exemple de réalisation possible auquel la présente invention s'applique, et il est entendu que la présente invention s'applique de manière plus générale à tout type de dispositif contenant au moins une dose.

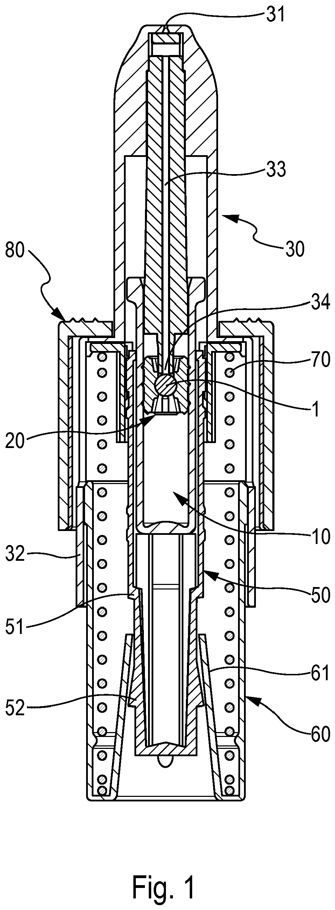

En se référant à la figure 1, le dispositif de distribution est un bidose qui comporte un réservoir 10 contenant deux doses de produit fluide. Un bouchon-piston 20 est monté coulissant dans ledit réservoir 10. Dans la position avant actionnement du dispositif, représentée sur la figure 1, ledit bouchon-piston 20 fait office de bouchon en isolant le contenu du réservoir 10.

Le bouchon-piston 20 comporte un manchon creux 26 pourvu d'une zone de réception centrale 21 disposée sensiblement au centre axial et radial dudit bouchon-piston 20, destinée à recevoir un élément d'obturation 1 en position d'obturation.

L'élément d'obturation 1 est de préférence sphérique, tel qu'une bille. Cet élément d'obturation 1 peut être réalisé en tout matériau approprié, tel que par exemple un matériau synthétique, un métal, une céramique ou du verre.

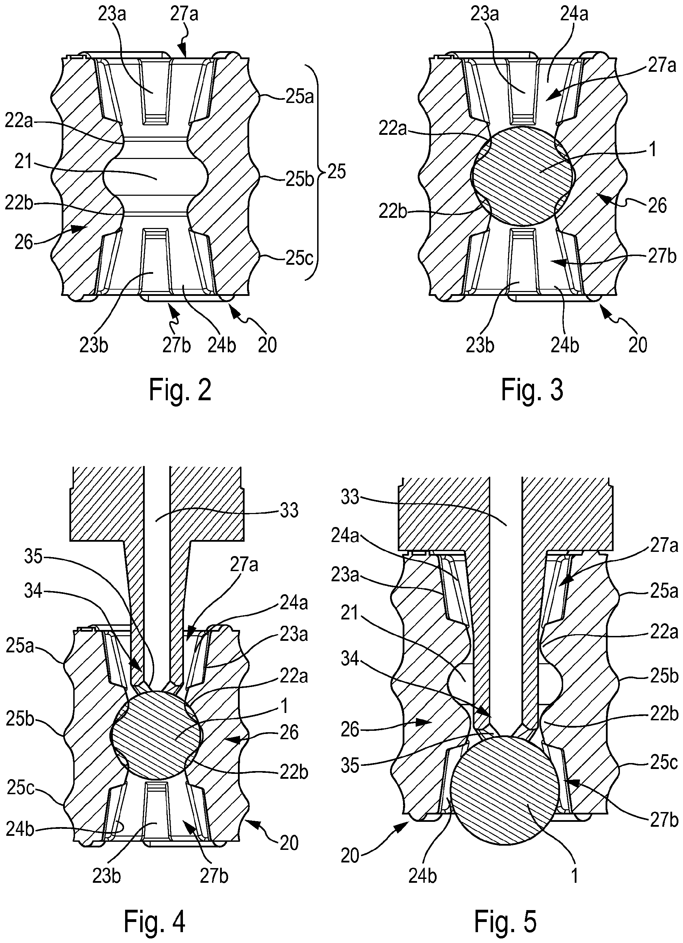

La zone de réception centrale 21 est définie entre un rétrécissement supérieur 22a et un rétrécissement inférieur 22b, de sorte que lorsque l'élément d'obturation 1 est disposé dans ladite zone de réception centrale 21, il y a deux surfaces périphériques d'étanchéité entre l'élément d'obturation 1 et le bouchon-piston 20, une première surface d'étanchéité étant formée par le rétrécissement supérieur 22a et la seconde surface d'étanchéité étant formée par le rétrécissement inférieur 22b, comme illustré sur les figures 3 et 4. L'étanchéité en position d'obturation de l'élément d'obturation 1 est donc garantie, et cette position d'obturation est particulièrement stable.

La zone de réception centrale 21 se prolonge axialement d'un côté par une zone de réception inférieure 27b. Avantageusement, la zone de réception centrale 21 se prolonge axialement du côté opposé par une zone de réception supérieure 27a, symétrique à la zone de réception inférieure 27b. Chaque zone de réception 27a, 27b a avantageusement une forme légèrement conique s'évasant radialement vers l'extérieur à partir de la zone de réception centrale 21. Chaque zone de réception 27a, 27b comporte des parties de paroi 24a, 24b séparées par des rainures radiales 23a, 23b s'étendant axialement. Dans l'exemple des figures, il y a quatre parties de paroi 24a, 24b et quatre rainures radiales 23a, 23b réparties sur la périphérie, mais on pourrait envisager un nombre quelconque de parties de paroi et de rainures radiales.

De par la forme conique s'évasant radialement vers l'extérieur à partir de la zone de réception centrale 21, le diamètre interne maximal desdites parties de paroi 24a, 24b se situe au niveau du bord axial extérieur de chaque zone de réception supérieure et inférieure 27a, 27b. De préférence, ledit diamètre interne maximal est sensiblement identique au diamètre externe maximal de l'élément d'obturation 1.

Lors de l'assemblage, l'élément d'obturation 1 est inséré à force dans la zone de réception centrale 21, en position d'obturation. Avantageusement, dans le mode de réalisation décrit, l'élément d'obturation 1 est d'abord disposé dans la zone de réception supérieure 27a, puis inséré à force dans la zone de réception centrale 21, en position d'obturation.

Lors de l'actionnement, l'élément d'obturation 1 est expulsé mécaniquement hors de la zone de réception centrale 21, comme cela sera décrit plus en détail ci-après, et il vient alors se positionner dans la zone de réception inférieure 27b, pour y être maintenu dans une position d'ouverture par lesdites parties de paroi 24b. Dans cette position d'ouverture, le produit fluide peut s'écouler autour dudit élément d'obturation 1 via les rainures radiales 23b. Le maintien de l'élément d'obturation 1 dans le piston-bouchon 20 après son expulsion de sa position d'obturation est avantageux en ce que cela permet de limiter le volume mort induit par la zone de réception inférieure 27b. Ceci permet aussi d'éviter tout risque de rebouchage du bouchon-piston 20.

Le bouchon-piston 20 comporte une partie externe d'étanchéité 25, formée sur la périphérie externe du manchon creux 26. Dans l'exemple des figures, cette partie d'étanchéité 25 comporte trois bourrelets périphériques, un bourrelet supérieur 25a, un bourrelet central 25b et un bourrelet inférieur 25c, qui réalisent l'étanchéité contre la paroi latérale interne du réservoir 10. Bien entendu, un nombre quelconque de bourrelets pourrait être prévu, par exemple un seul bourrelet, deux bourrelets ou plus de trois bourrelets.

En position d'obturation de l'élément d'obturation 1, celui-ci exerce une force radiale sur les rétrécissements supérieur et inférieur 22a, 22b, qui va au moins partiellement être transmise radialement à ladite partie externe d'étanchéité 25, ce qui va augmenter l'interaction et donc l'étanchéité entre le bouchon-piston 20 et le réservoir 10. Lors de l'actionnement, lorsque l'élément d'obturation 1 est expulsé de sa position d'obturation, cette force radiale diminue voire disparait, et par conséquent, l'interaction entre le bouchon-piston 20 et le réservoir 10 diminue également, ce qui facilite le déplacement du bouchon-piston 20 dans le réservoir 10 lors de l'actionnement. Avantageusement, le déplacement du bouchon-piston 20 dans le réservoir 10 lors de l'actionnement ne débute qu'après expulsion de l'élément d'obturation 1 de sa position d'obturation.

Comme visible sur les figures, le bouchon-piston 20 est avantageusement parfaitement symétrique et donc utilisable indifféremment dans un sens ou dans l'autre. Ce caractère réversible du bouchon-piston 20 est avantageux, notamment lors de l'assemblage. En effet, il n'est pas nécessaire d'orienter le bouchon-piston 20 dans un sens particulier, de sorte que l'assemblage du bouchon-piston 20 est simplifié et donc moins coûteux.

Une tête de distribution 30 est assemblée sur ledit réservoir 10 en étant déplaçable axialement par rapport à celui-ci. En particulier, un déplacement axial de la tête de distribution 30 par rapport au réservoir 10 provoque le déplacement du bouchon-piston 20 dans le réservoir 10 et ainsi la distribution du produit fluide contenu dans ledit réservoir. La tête de distribution 30 comporte un canal de distribution 33 qui mène depuis une pointe d'ouverture 34 jusqu'à un orifice de distribution 31 de la tête de distribution 30. Cette pointe d'ouverture 34 coopère lors de l'actionnement avec l'élément d'obturation 1 pour le déplacer de sa position d'obturation vers sa position d'ouverture. En amont de l'orifice de distribution 31, il peut être prévu un profil de pulvérisation, qui peut être d'un quelconque type connu et qui n'est pas représenté plus en détail sur les dessins, pour distribuer le produit fluide sous forme de spray.

La pointe d'ouverture 34 peut comporter à son extrémité axiale une ou plusieurs ouvertures 35, tels que des trous ou des fentes, qui permettent le passage de produit fluide lorsque la pointe d'ouverture 34 est en contact de l'élément d'obturation 1. En variante, l'extrémité de ladite pointe d'ouverture 34 pourrait avoir une forme appropriée pour laisser passer le produit fluide lors de l'actionnement.

Dans l'exemple représenté, le réservoir 10 est fixé dans un corps 50, qui est donc solidaire dudit réservoir 10, et qui se déplace ensemble avec lui.

La tête de distribution 30 comporte une jupe latérale inférieure 32 adaptée à coopérer avec un organe d'actionnement 60. Un élément reposedoigts 80 est assemblé autour de ladite tête de distribution 30, ou en variante il peut être formé de manière monobloc avec elle.

Ledit organe d'actionnement 60 est axialement déplaçable à l'intérieur de ladite jupe latérale 32 de la tête de distribution 30 pour réaliser les actionnements successifs de dispositif. A cet effet, l'organe d'actionnement 60 comporte au moins une patte inclinée 61 qui est adaptée à coopérer avec des projections 51, 52 du corps 50 pour réaliser les actionnements successifs.

Un ressort de rappel 70 est monté entre l'organe d'actionnement 60 et la tête de distribution 30, pour ramener ledit organe d'actionnement 60 dans sa position de départ après chaque actionnement.

Typiquement, la force axiale nécessaire pour expulser l'élément d'obturation 1 de sa position d'obturation est d'environ 2 à 8 N, avantageusement de 4 à 6 N. La force axiale nécessaire pour déplacer le bouchon-piston 20 dans le réservoir 10 avec l'élément d'obturation 1 en position d'obturation est typiquement comprise entre environ 10 et 15 N. Ainsi, l'expulsion de l'élément d'obturation 1 de sa position d'obturation ne va pas déplacer le bouchon-piston 20, ce déplacement ne commençant que lorsque l'élément d'obturation 1 est dans sa position d'ouverture. Après expulsion de l'élément d'obturation de sa position d'obturation, la force nécessaire pour déplacer le bouchon-piston 20 diminue, typiquement entre 2 et 10 N, ce qui facilite l'actionnement pour l'utilisateur. La présente invention permet donc de renforcer l'étanchéité au repos, tout en facilitant l'actionnement du dispositif. Par ailleurs, du fait que la force nécessaire pour expulser l'élément d'obturation 1 de sa position d'obturation est inférieure à la force nécessaire pour déplacer le bouchon-piston 20 dans le réservoir 10 avec l'élément d'obturation 1 en position d'obturation, il n'y a pas de risque de surcompression du produit fluide, et donc la distance parcourue par le bouchon-piston 20 correspond au volume de dose expulsé, ce qui dans le cas d'un bidose permet un bon équilibre de volume entre les deux doses.

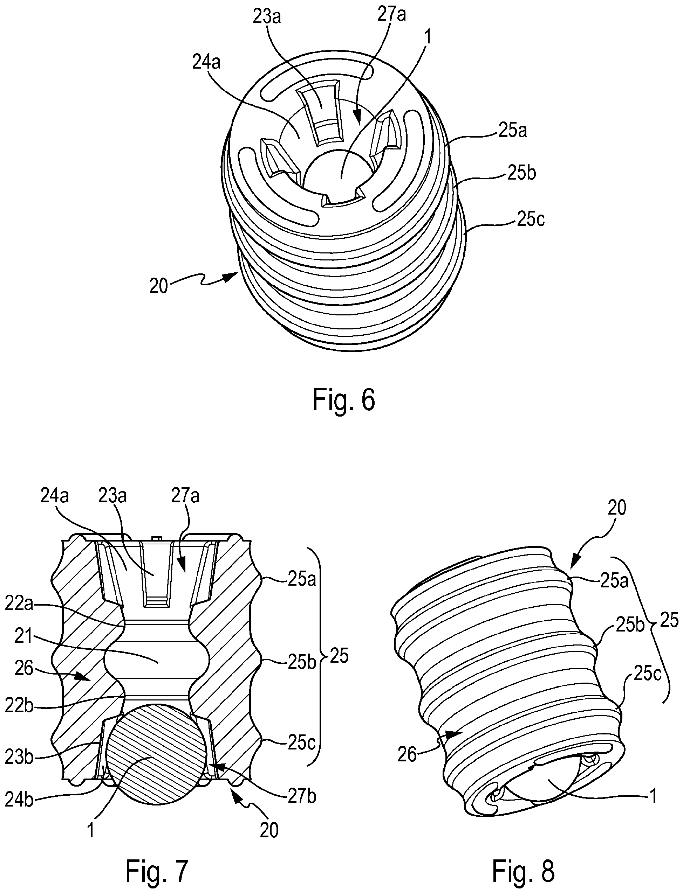

Le fonctionnement du dispositif représenté sur la figure 1 est le suivant. Dans la position de repos de la figure 1, le bouchon-piston 20 isole le contenu du réservoir 10 de l'atmosphère. Lorsque l'utilisateur appuie simultanément sur le repose-doigt 80 et sur l'organe d'actionnement 60, il va déplacer ledit organe d'actionnement 60 à l'intérieur de la jupe latérale 32 de la tête de distribution 30. Ceci va pousser le corps 50 axialement vers le haut à partir de la position représentée sur la figure 1, par l'intermédiaire des pattes 61 qui vont pousser sur l'épaulement 51 dudit corps. Ceci va comprimer le ressort 70 et déplacer le réservoir 10 par rapport à la tête de distribution 30. Lorsque le réservoir 10 va commencer à se déplacer par rapport à la tête de distribution 30, la pointe d'ouverture 34 du canal de distribution 33 va venir expulser l'élément d'obturation 1 de sa position d'obturation, visible sur les figures 1, 3, 4 et 6, vers sa position d'ouverture, visible sur les figures 5, 7 et 8, pour faire communiquer l'intérieur du réservoir 10 avec ledit canal d'expulsion 33. Une poursuite de l'actionnement va provoquer un déplacement du bouchon-piston 20 à l'intérieur du réservoir 10 et donc une distribution d'une première dose. Le produit fluide est donc poussé par ledit piston 20 autour de l'élément d'obturation 1, à travers la pointe d'ouverture 34 dans le canal de distribution 33, puis hors du dispositif à travers l'orifice de distribution 31.

Après distribution de la première dose, lorsque l'utilisateur relâche l'organe d'actionnement 60, le ressort 70 va ramener celui-ci vers sa position de départ. Pendant ce retour de l'organe d'actionnement 60, le réservoir 10 et le corps 50 ne reviennent pas en arrière, car ces deux composants restent maintenus dans ladite tête de distribution 30. Eventuellement, la tête de distribution 30 peut comporter des moyens anti-retour pour empêcher un retour dudit corps 50 et/ou dudit réservoir 10 en arrière. Lorsque l'organe d'actionnement 60 revient vers sa position de repos, les pattes 61 vont se positionner sous la deuxième projection 52 du corps 50 ce qui va permettre à l'utilisateur d'actionner une deuxième fois le dispositif pour distribuer la deuxième dose de produit fluide.

Bien entendu, la présente invention a été décrite en référence à un mode de réalisation, qui n'est pas limitatif, et toute modification utile peut être apportée à la présente invention sans sortir du cadre de celle-ci tel que défini par les revendications annexées.

## Revendications

1. Dispositif de distribution de produit fluide comportant un réservoir (10) contenant au moins une dose de produit fluide, un bouchon-piston (20), monté coulissant dans ledit réservoir (10) pour distribuer du produit fluide, une tête de distribution (30), pourvue d'un orifice de distribution (31), axialement déplaçable par rapport audit réservoir (10) pour déplacer ledit bouchon-piston (20) dans ledit réservoir (10) pour ainsi distribuer du produit fluide à travers ledit orifice de distribution (31), ledit bouchon-piston (20) comportant un manchon creux (26) pourvu d'une zone de réception centrale (21) disposée sensiblement au centre axial et radial dudit bouchon-piston (20), un élément d'obturation (1) étant disposé dans ladite zone de réception centrale (21) en position d'obturation et étant lors de l'actionnement expulsé de ladite position d'obturation vers une position d'ouverture, **caractérisé en ce que** ladite zone de réception centrale (21) se prolonge axialement d'un côté par une zone de réception inférieure (27b) comportant au moins deux parties de paroi (24b) séparées par au moins deux rainures radiales (23b) s'étendant axialement, lesdites parties de paroi (24b) de ladite zone de réception inférieure (27b) retenant ledit élément d'obturation (1) dans sa position d'ouverture, avec le produit fluide pouvant lors de l'actionnement s'écouler depuis ledit réservoir (10) autour dudit élément d'obturation (1) en position d'ouverture à travers lesdites rainures radiales (23b) vers ledit orifice de distribution (31).

2. Dispositif selon la revendication 1, dans lequel ledit bouchon-piston (20) comporte une zone de réception supérieure (27a) comportant au moins deux parties de paroi (24a) séparées par au moins deux rainures radiales (23a) s'étendant axialement, ladite zone de réception supérieure (27a) étant symétrique par rapport à ladite zone de réception inférieure (27b), de sorte que ledit bouchon-piston (20) est symétrique et utilisable indifféremment dans un sens ou dans l'autre.

3. Dispositif selon la revendication 2, dans lequel chaque zone de réception (27a, 27b) a une forme légèrement conique s'évasant radialement vers l'extérieur à partir de la zone de réception centrale (21).

4. Dispositif selon la revendication 3, dans lequel le diamètre interne maximal desdites parties de paroi (24a, 24b) se situe au niveau du bord axial extérieur de chaque zone de réception supérieure et inférieure (27a, 27b), ledit diamètre interne maximal étant sensiblement identique au diamètre externe maximal dudit élément d'obturation (1).

5. Dispositif selon l'une quelconque des revendications 2 à 4, dans lequel chaque zone de réception (27a, 27b) comporte quatre parties de paroi (24a, 24b) et quatre rainures radiales (23a, 23b) réparties sur la périphérie.

6. Dispositif selon l'une quelconque des revendications précédentes, dans lequel ledit bouchon-piston (20) comporte une partie externe d'étanchéité (25), formée sur la périphérie externe dudit manchon creux (26), ladite partie d'étanchéité (25) comportant au moins un bourrelet périphérique (25a, 25b, 25c) qui réalise l'étanchéité contre une paroi latérale interne dudit réservoir (10).

7. Dispositif selon la revendication 6, dans lequel ledit élément d'obturation (1), en position d'obturation, exerce une force radiale sur ladite partie externe d'étanchéité (25), augmentant l'interaction entre ledit bouchon-piston (20) et ledit réservoir (10) et donc l'étanchéité, et lors de l'actionnement, lorsque ledit élément d'obturation (1) est expulsé de sa position d'obturation, l'interaction entre ledit bouchon-piston (20) et ledit réservoir (10) diminue, facilitant ainsi le déplacement dudit bouchon-piston (20) dans ledit réservoir (10) lors de l'actionnement.

8. Dispositif selon l'une quelconque des revendications précédentes, dans lequel ledit élément d'obturation (1) est sphérique, tel qu'une bille.

9. Dispositif selon l'une quelconque des revendications précédentes, dans lequel ladite zone de réception centrale (21) est définie entre un rétrécissement supérieur (22a) et un rétrécissement inférieur (22b), de sorte qu'en position d'obturation, il y a deux surfaces périphériques d'étanchéité entre ledit élément d'obturation (1) et ledit bouchon-piston (20).

10. Dispositif selon l'une quelconque des revendications précédentes, dans lequel ladite tête de distribution (30) comporte un canal de distribution (33) reliant une pointe d'ouverture (34) audit orifice de distribution (31), ladite pointe d'ouverture (34) coopérant lors de l'actionnement avec ledit élément d'obturation (1) pour le déplacer de sa position d'obturation vers sa position d'ouverture, ladite pointe d'ouverture (34) comportant à son extrémité axiale une ou plusieurs ouvertures (35), tels que des trous ou des fentes, qui permettent le passage de produit fluide lorsque ladite pointe d'ouverture (34) est en contact avec ledit élément d'obturation (1).

11. Dispositif selon l'une quelconque des revendications précédentes, dans lequel ledit réservoir (10) contient deux doses de produit fluide, distribuées lors de deux actionnements successifs.

12. Dispositif selon l'une quelconque des revendications 1 à 10, dans lequel ledit réservoir (10) contient une seule dose de produit fluide, distribuée lors d'un seul actionnement.

## Patentansprüche

1. Vorrichtung zur Abgabe eines Fluidprodukts, umfassend ein Reservoir (10), das zumindest eine Dosis an Fluidprodukt enthält, einen Kolbenstopfen (20), der gleitbeweglich in dem Reservoir (10) gelagert ist, um das Fluidprodukt abzugeben, einen mit einer Abgabeöffnung (31) versehenen Abgabekopf (30), der axial in Bezug auf das Reservoir (10) verschiebbar ist, um den Kolbenstopfen (20) in dem Reservoir (10) zu verschieben, um so Fluidprodukt durch die Abgabeöffnung (31) abzugeben, wobei der Kolbenstopfen (20) eine hohle Hülse (26) umfasst, die mit einem zentralen Aufnahmebereich (21) versehen ist, der im Wesentlichen axial und radial mittig im Kolbenstopfen (20) angeordnet ist, wobei in dem zentralen Aufnahmebereich (21) ein Verschlusselement (1) in Verschlussstellung angeordnet ist und bei Betätigung aus der Verschlussstellung in eine Öffnungsstellung gedrückt wird,
**dadurch gekennzeichnet, dass** sich der zentrale Aufnahmebereich (21) auf einer Seite axial mit einen unteren Aufnahmebereich (27b) fortsetzt, der zumindest zwei Wandabschnitte (24b) aufweist, die durch zumindest zwei sich axial erstreckende radiale Nuten (23b) getrennt sind, wobei die Wandabschnitte (24b) des unteren Aufnahmebereichs (27b) das Verschlusselement (1) in seiner Öffnungsstellung halten, wobei das Fluidprodukt bei Betätigung aus dem Reservoir (10) um das in Öffnungsstellung befindliche Verschlusselement (1) herum durch die radialen Nuten (23b) zu der Abgabeöffnung (31) fließen kann.

2. Vorrichtung nach Anspruch 1,
wobei der Kolbenstopfen (20) einen oberen Aufnahmebereich (27a) mit zumindest zwei Wandabschnitten (24a) aufweist, die durch zumindest zwei sich axial erstreckende radiale Nuten (23a) getrennt sind, wobei der obere Aufnahmebereich (27a) symmetrisch zum unteren Aufnahmebereich (27b) verläuft, so dass der Kolbenstopfen (20) symmetrisch ausgeführt und beliebig in der einen oder anderen Richtung verwendbar ist.

3. Vorrichtung nach Anspruch 2,
wobei jeder Aufnahmebereich (27a, 27b) eine leicht konische Form hat, die sich von dem zentralen Aufnahmebereich (21) radial nach außen erweitert.

4. Vorrichtung nach Anspruch 3,
wobei der maximale Innendurchmesser der Wandabschnitte (24a, 24b) jeweils an der äußeren axialen Kante des oberen und des unteren Aufnahmebereichs (27a, 27b) liegt, wobei der maximale Innendurchmesser im Wesentlichen gleich dem maximalen Außendurchmesser des Verschlusselements (1) ist.

5. Vorrichtung nach einem der Ansprüche 2 bis 4,
wobei jeder Aufnahmebereich (27a, 27b) vier Wandabschnitte (24a, 24b) und vier über den Umfang verteilt angeordnete radiale Nuten (23a, 23b) aufweist.

6. Vorrichtung nach einem der vorhergehenden Ansprüche,
wobei der Kolbenstopfen (20) einen äußeren Dichtungsabschnitt (25) aufweist, der am Außenumfang der hohlen Hülse (26) ausgebildet ist, wobei der Dichtungsabschnitt (25) zumindest einen Umfangswulst (25a, 25b, 25c) aufweist, der gegen eine innere Seitenwand des Reservoirs (10) abdichtet.

7. Vorrichtung nach Anspruch 6,
wobei das Verschlusselement (1) in der Verschlussstellung eine radiale Kraft auf den äußeren Dichtungsabschnitt (25) ausübt, wodurch die Wechselwirkung zwischen dem Kolbenstopfen (20) und dem Reservoir (10) und damit die Abdichtung erhöht wird, und bei Betätigung, wenn das Verschlusselement (1) aus seiner Verschlussstellung gedrückt wird, die Wechselwirkung zwischen dem Kolbenstopfen (20) und dem Reservoir (10) abnimmt, wodurch die Verschiebung des Kolbenstopfens (20) in dem Reservoir (10) bei der Betätigung erleichtert wird.

8. Vorrichtung nach einem der vorhergehenden Ansprüche,
wobei das Verschlusselement (1) sphärisch, wie etwa eine Kugel, ausgebildet ist.

9. Vorrichtung nach einem der vorhergehenden Ansprüche,
wobei der zentrale Aufnahmebereich (21) zwischen einer oberen Verengung (22a) und einer unteren Verengung (22b) definiert ist, so dass in der Verschlussstellung zwei umlaufende Dichtflächen zwischen dem Verschlusselement (1) und dem Kolbenstopfen (20) vorliegen.

10. Vorrichtung nach einem der vorhergehenden Ansprüche,
wobei der Abgabekopf (30) einen Abgabekanal (33) aufweist, der eine Öffnungsspitze (34) mit der Abgabeöffnung (31) verbindet, wobei die Öffnungsspitze (34) bei der Betätigung mit dem Verschlusselement (1) zusammenwirkt, um es aus seiner Verschlussstellung in seine Öffnungsstellung zu bewegen, wobei die Öffnungsspitze (34) an ihrem axialen Ende eine oder mehrere Öffnungen (35), wie Löcher oder Schlitze, aufweist, die den Durchtritt des Fluidprodukts ermöglichen, wenn die Öffnungsspitze (34) mit dem Verschlusselement (1) in Kontakt ist.

11. Vorrichtung nach einem der vorhergehenden Ansprüche,
wobei das Reservoir (10) zwei Dosen an Fluidprodukt enthält, die bei zwei aufeinanderfolgenden Betätigungen abgegeben werden.

12. Vorrichtung nach einem der Ansprüche 1 bis 10,
wobei das Reservoir (10) eine Einzeldosis an Fluidprodukt enthält, die bei einer Einzelbetätigung abgegeben wird.

## Claims

1. A fluid product dispensing device comprising a reservoir (10) containing at least one dose of fluid product, a piston-stopper(20) mounted with the ability to slide in said reservoir (10) in order to dispense fluid product, a dispensing head (30), provided with a dispensing orifice (31), and able to move axially with respect to said reservoir (10) so as to move said piston-stopper (20) in said reservoir (10) in order thus to dispense the fluid product through said dispensing orifice (31), said piston-stopper (20) comprising a hollow sleeve (26) provided with a central receiving zone (21) arranged substantially at the axial and radial centre of said piston plug (20), a shut-off element (1) being positioned in said central receiving zone (21) in a shutting-off position and being, during actuation, expelled from said shutting-off position towards an open position, **characterized in that** said central receiving zone (21) is extended axially on one side by a lower receiving zone (27b) comprising at least two wall parts (24b) separated by at least two axially extending radial grooves, said wall parts (24b) of said lower receiving zone (27b) holding said shut-off element (1) in its open position, with the fluid product being able, during actuation, to flow from said reservoir (10) around said shut-off element (1) in the open position via said radial grooves (23b) towards said dispensing orifice (31).

2. The device according to claim 1, wherein said piston-stopper (20) comprises an upper receiving zone (27a) comprising at least two wall parts (24a) separated by at least two radial grooves (23a) extending axially, said upper receiving zone (27a) being symmetrical relative to said lower receiving zone (27b), such that said piston-stopper (20) is symmetrical and can be used equally well in one direction or the other.

3. The device according to claim 2, wherein each reception zone (27a, 27b) has a slightly conical shape flaring radially outwards from the central reception zone (21).

4. The device according to claim 3, wherein the maximum inside diameter of said wall parts (24a, 24b) is situated at the outer axial edge of each upper and lower receiving zone (27a, 27b), said maximum inside diameter being substantially identical to the maximum outside diameter of said shut-off element (1).

5. The device according to any one of claims 2 to 4, wherein each receiving zone (27a, 27b) comprises four wall parts (24a, 24b) and four radial grooves (23a, 23b) distributed over the periphery.

6. The device according to any one of the preceding claims, wherein said piston-stopper (20) comprises an outer sealing part (25), formed on the outer periphery of said hollow sleeve (26), said sealing part (25) comprising at least one peripheral bead (25a, 25b, 25c) which provides sealing against an inner side wall of said reservoir (10).

7. The device according to claim 6, wherein said shut-off element (1), in the shutting-off position, exerts a radial force on said external sealing part (25), increasing the interaction between said piston-stopper (20) and said reservoir (10) and therefore the sealing, and during actuation, when said shut-off element (1) is expelled from its shutting-off position, the interaction between said piston-stopper (20) and said reservoir (10) decreases, thus facilitating the movement of said piston-stopper (20) in said reservoir (10) during actuation.

8. The device according to any one of the preceding claims, wherein said shut-off element (1) is spherical, such as a ball.

9. The device according to any one of the preceding claims, wherein said central receiving zone (21) is defined between an upper narrowing (22a) and a lower narrowing (22b), such that in the shutting-off position, there are two peripheral sealing surfaces between said sealing element (1) and said piston-stopper (20).

10. The device according to any one of the preceding claims, wherein said dispensing head (30) includes a dispensing channel (33) that connects an opening tip (34) to said dispensing orifice (31), said opening tip (34) co-operating with said shut-off element (1) during actuation so as to move it from its shutting-off position to its open position, said opening tip (34) including one or more openings (35) at its axial end, such as holes or slots, that enable fluid product to pass when said opening tip (34) is in contact with said shut-off element (1).

11. The device according to any one of the preceding claims, wherein said reservoir (10) contains two doses of fluid product, dispensed in two successive actuations.

12. The device according to any one of the preceding claims, wherein said reservoir (10) contains one single dose of fluid product, dispensed in one single actuation.
